# EUROPÄISCHE PATENTANMELDUNG

(11) **EP 3 985 407 A1**
(43) Veröffentlichungstag der Anmeldung: **20.04.2022**
(21) Anmeldenummer: 21162956.3
(22) Anmeldetag: 16.03.2021
(51) Int. Cl.: G01R 33/28, A61B 90/00

(54) **MARKEN FÜR EIN MAGNETRESONANZTOMOGRAPHIESYSTEM UND KORRESPONDIERENDE BILDAKQUISITION**

(71) Anmelder: Siemens Healthcare GmbH, 91052 Erlangen (DE)
(72) Erfinder: Grodzki, David, 91058 Erlangen (DE)

(57) **Zusammenfassung**

Marker für Magnetresonanztomographiesysteme sollen interessierende Bildanteile weniger stören. Dazu wird ein Verfahren zum Herstellen eines Markers (1) vorgeschlagen, bei dem eine Feldstärke für die Akquisition, eine Auslesebandbreite für die Akquisition und eine Größe sowie ein Material für einen Markerkern (2) vorgegeben werden. Eine Signalveränderung wird gegenüber dem nicht vorhandenen Marker bei der Akquisition mit der vorgegebenen Feldstärke und der vorgegebenen Bandbreite um den Markerkern (2) herum ermittelt. Zudem wird eine Ausdehnungsgrenze (7) ermittelt, innerhalb der die Signalveränderung einen vorgegebenen Betrag überschreitet. Schließlich wird die Markerhülle (3) des Markers (1) um den Markerkern (2) derart dimensioniert, dass die Ausdehnungsgrenze (7) zumindest bereichsweise innerhalb der Markerhülle (3) liegt. Bei der Bildakquisition kann ein Mustererkennungsalgorithmus eingesetzt werden, der auf die genannten Parameter abgestimmt ist.

## Beschreibung

Die vorliegende Erfindung betrifft ein Verfahren zum Herstellen eines Markers für ein Magnetresonanztomographiesystem. Darüber hinaus betrifft die vorliegende Erfindung einen entsprechenden Marker für eine Akquisition mittels eines Magnetresonanztomographiesystems bei einer vorgegebenen Feldstärke und vorgegebenen Auslesebandbreite, wobei der Marker eine Markerhülle und einen Markerkern vorgegebener Größe und vorgegebenen Materials, welcher vollständig von der Markerhülle umgeben ist, aufweist. Ferner betrifft die vorliegende Erfindung ein Verfahren zur Akquisition eines Bildes mittels eines Magnetresonanztomographiesystems durch Empfangen von MR-Signalen. Des Weiteren betrifft die vorliegende Erfindung ein entsprechendes Computerprogrammprodukt.

Magnetresonanztomographen bzw. Magnetresonanztomographiesysteme sind bildgebende Vorrichtungen, die zur Abbildung eines Untersuchungsobjektes Kernspins des Untersuchungsobjektes mit einem starken äußeren Magnetfeld ausrichten und durch ein magnetisches Wechselfeld zur Präzession um diese Ausrichtung anregen. Die Präzession bzw. Rückkehr des Spins aus diesem angeregten in einen Zustand mit geringerer Energie wiederum erzeugt als Antwort ein magnetisches Wechselfeld, das über Antennen empfangen wird.

Mithilfe von magnetischen Gradientenfeldern wird den Signalen eine Ortskodierung aufgeprägt, die nachfolgend eine Zuordnung von dem empfangenen Signal zu einem Volumenelement ermöglicht. Das empfangene Signal wird dann ausgewertet und eine dreidimensionale bildgebende Darstellung des Untersuchungsobjekts bereitgestellt. Zum Empfang des Signals werden vorzugsweise lokale Empfangsantennen, sogenannte Lokalspulen, verwendet, die zur Erzielung eines besseren Signal-Rauschabstandes unmittelbar am Untersuchungsobjekt angeordnet werden. Die Empfangsantennen können auch in einer Patientenliege verbaut sein.

In vielen Anwendungen der medizinischen Bildgebung ist das Sichtbarmachen und Verfolgen punktförmiger Objekte, im Folgenden "Seeds" bzw. "Marker" genannt, von Interesse. Die Anwendungen reichen vom Nachverfolgen von Bewegungen dieser Seeds durch den Körper bis zur Markierung von bestimmten Stellen im Körper bei Folgeuntersuchungen oder Untersuchungen, in denen eine bestimmte Stelle über die Untersuchung hinweg schnell und einfach wieder auffindbar sein soll.

Zudem sind Verfahren bekannt, in denen die Bewegung von Seeds zur Bewegungskorrektur oder Bewegungsüberwachung eines Objektes genutzt wird. Als Seeds können etwa Kügelchen, beispielsweise mit einem Durchmesser in der Größenordnung von 5mm verwendet werden, die mit einer MR-aktiven Flüssigkeit, wie z. B. Kontrastmittel, gefüllt sind. Die Kügelchen werden an solchen Stellen platziert, an denen sich die zu beobachtende relative oder absolute Bewegung feststellen lässt, z. B. bei der Zahnbildgebung auf einer Schiene am Ober- und Unterkiefer, oder indem sie zwischen Backe und Zahn platziert werden. Erstrebenswert dabei wäre, dass die Bilder der Zähne durch die Seeds nicht gestört sind.

Als Seeds können je nach Anwendung und benötigter Größe unterschiedliche Lösungen gewählt werden. So ist z. B. bekannt, dass mit Gadolinium, Öl oder Vitaminen gefüllte Kügelchen (z. B. Vitamin B- oder Fischöl-Kügelchen aus der Apotheke) mit Durchmessern von ca. 0,5cm in T1-gewichteten Messungen besonders hell aufleuchten. So sind sie auf einer 2D- oder 3D-Aufnahme durch ein relativ hohes CNR (Kontrast-zu-Rauschverhältnis) sichtbar. Stoffe mit ähnlichen Relaxationszeiten leuchten jedoch genau so hell auf, so dass eine automatisierte Erkennung nicht immer trivial ist.

Im Stand der Technik sind auch sehr kleine, magnetisch gelabelte Seeds (wesentlich <lmm) bekannt, die durch Ausnutzen der Suszeptibilitätswirkung bzw. Störung des Hauptmagnetfeldes sichtbar gemacht werden können, so dass im Bild annähernd nur die Bereiche um die Seeds aufleuchten. In diese Seeds können beispielsweise Zellen eingebracht werden. Auch ist bekannt, dass solche Seeds mit anderen MR-sichtbaren Stoffen wie Fluor-19 gefüllt sind, so dass sie in einer Multi-Nuklei-Messung identifiziert und lokalisiert werden können.

Für größere (ca. 0,1 bis 0,5cm Durchmesser) Seeds sind jedoch keine Lösungen bekannt, mit denen diese sichtbar gemacht werden können, ohne beispielsweise im Fall von Metallkügelchen sehr großflächige, weit über die Abmessungen des Seeds hinausreichende Störungen hervorzurufen. Dies wäre insbesondere bei Seeds, die z. B. unmittelbar an einem Zahn anliegen, nicht hilfreich, wenn dieser Zahn abgebildet werden soll.

Die Aufgabe der vorliegenden Erfindung besteht somit darin, ein Verfahren zur Herstellung eines Seeds bzw. Markers anzugeben, das zu Markern führt, welche die abzubildenden Objekte weniger stören. Darüber hinaus soll ein Bild-Akquisitionsverfahren angegeben werden, bei dem derartige Marker besser erkannt werden können.

Erfindungsgemäß wird diese Aufgabe gelöst durch die Gegenstände der unabhängigen Ansprüche. Vorteilhafte Weiterbildungen der Erfindung ergeben sich aus den Unteransprüchen.

Entsprechend der vorliegenden Erfindung wird demnach ein Verfahren zum Herstellen eines Markers für ein Magnetresonanztomographiesystem bereitgestellt. Derartige Marker werden vielfach auch "Seeds" oder "Phantome" genannt. Das Magnetresonanztomographiesystem kann zur Bildgewinnung bei medizinischen Gewebeuntersuchungen, aber auch bei technischen Materialüberprüfungen eingesetzt werden. Die Marker können dabei dazu dienen, bestimmte Positionen des zu untersuchenden Objekts zu markieren und/oder gegebenenfalls entsprechende Bewegungen nachzuverfolgen bzw. zu kompensieren. Generell können aber auch andere Anwendungszwecke in Frage kommen.

Bei dem erfindungsgemäßen Verfahren wird für die Akquisition mit dem Magnetresonanztomographiesystem eine Feldstärke vorgegeben. Dies bedeutet, dass es für das Herstellen des Markers von wesentlicher Bedeutung ist, für welche Feldstärke er verwendet werden soll. Hierbei muss nicht eine einzelne Feldstärke vorgegeben werden, sondern vielmehr kann ein Feldstärkebereich vorgegeben werden, in dem eine bestimmte Feldstärke liegt.

Darüber hinaus wird für die Akquisition mit dem Magnetresonanztomographiesystem eine Auslesebandbreite vorgegeben. Diese Auslesebandbreite ist deswegen von Bedeutung, weil die räumliche Auswirkung von MR-Signalen z. B. umgekehrt proportional zur Auslesebandbreite ist. So können sich beispielsweise bei geringer Auslesebandbreite Störungen räumlich weiter bemerkbar machen als bei hohen Auslesebandbreiten. Die Auslesebandbreite kann beispielsweise zwischen 200 und 500 Hz / Pixel liegen.

Ferner wird für das erfindungsgemäße Herstellungsverfahren eine Größe und ein Material für einen Markerkern des Markers vorgegeben. Der Marker besteht aus einem Markerkern und einer Markerhülle. Der Markerkern sollte aus einem MR-aktiven Material bestehen, wie es einleitend beschrieben wurde. Für die Herstellung des Markers ist neben dem Material natürlich auch die Größe des Markerkerns von wesentlicher Bedeutung. Je größer der Marker ist, desto leichter ist er auf einem Bild zu erkennen, aber desto mehr verdeckt er unter Umständen auch von dem zu untersuchenden Objekt.

Um nun den Marker geeignet dimensionieren zu können, erfolgt ein Ermitteln einer Signalveränderung gegenüber dem nicht vorhandenen Marker bei der Akquisition mit der vorgegebenen Feldstärke und der vorgegebenen Bandbreite um den Markerkern herum. Die Signalveränderung bedeutet also diejenige Veränderung des Signals um den Markerkern herum, die der Markerkern selbst hervorruft. D. h. der Markerkern strahlt quasi in seine Umgebung ab. Wäre der Markerkern nicht vorhanden bzw. MRneutral, so ergäbe sich im Bereich des (fiktiven) Markerkerns ein Grundsignal. Gegenüber diesem Grundsignal ruft der Markerkern eine Signalveränderung, d. h. eine Signalerhöhung (hyperintense Veränderung) oder eine Signalverminderung (hypointense Veränderung) hervor. Beispielsweise kann das Grundsignal durch eine Referenzaufnahme ohne den Marker an der gewünschten Stelle ermittelt werden. Alternativ kann das Grundsignal aber auch durch ein Signal geschätzt werden, das in ausreichender Entfernung von dem Markerkern detektiert wird.

In einem weiteren Schritt des erfindungsgemäßen Verfahrens erfolgt ein Ermitteln einer Ausdehnungsgrenze um den Markerkern herum, innerhalb der die Signalveränderung einen vorgegebenen Betrag überschreitet. Die Signalveränderungen um den Markerkern nehmen häufig die Form von Keulen an. Dabei nimmt der Betrag der Signalveränderung in den Außenbereichen mit steigendem Abstand vom Markerkern ab. In einem bestimmten Abstand vom Markerkern sinkt die Signalveränderung also unter einen vorgegebenen Betrag. Dieser vorgegebene Betrag kann beispielsweise auf 10%, 20% oder einen anderen beliebigen Prozentwert festgelegt werden. Der vorgegebene Betrag legt also beispielsweise in einem 2D-Bild die Ausdehnungsgrenze fest, innerhalb der die Signalveränderungen durch den Markerkern größer als der festgelegte Betrag und außerhalb der die Signalveränderungen durch den Markerkern kleiner bzw. gleich dem festgelegten Betrag sein können.

Schließlich kann die Markerhülle des Markers um den Markerkern derart dimensioniert werden, dass die Ausdehnungsgrenze zumindest bereichsweise innerhalb der Markerhülle liegt. Wenn der Marker beispielsweise die Form einer Kugel besitzt, befindet sich die Ausdehnungsgrenze zumindest bereichsweise innerhalb der Kugel. Günstigerweise befindet sich die Ausdehnungsgrenze größtenteils oder vollständig innerhalb der Markerhülle. Falls es sich also bei dem Marker um eine Kugel handelt, kann die Markerhülle durch den Kugelradius dimensioniert werden, wenn sich der Markerkern im Zentrum befindet. Falls der Marker beispielsweise ellipsoidförmig gestaltet ist, können bei der Dimensionierung der Markerhülle die Halbachsen des Ellipsoids entsprechend festgelegt werden. Ähnliches gilt für andere geometrische Körper, die der Marker annehmen kann. Wesentlich ist nur, dass sich die Ausdehnungsgrenze zumindest bereichsweise, vorzugsweise vollständig, innerhalb der Markerhülle befindet.

In vorteilhafter Weise kann mit einem solchen Marker erreicht werden, dass ein abzubildendes Objekt nicht durch Artefakte des Markerkerns gestört wird. Vielmehr klingen die Störungsartefakte in der Markerhülle ab. Wenn also beispielsweise der Marker an einem Zahn anliegt, wird das Bild des Zahns durch den Markerkern bzw. den Marker nicht gestört. Die Markerhülle dient also als Abstandshalter zwischen dem abzubildenden Objekt und dem Markerkern.

In einer vorteilhaften Ausgestaltung ist vorgesehen, dass ein Material für die Markerhülle, die den Markerkern vollständig umgibt, vorgegeben und bei der Ermittlung der Signalveränderung berücksichtigt wird. Grundsätzlich ist es nicht notwendig, dass das Material der Markerhülle für das Ermitteln bzw. Schätzen einer Signalveränderung bei der Bildakquisition bekannt sein muss. Vielmehr kann auch von einem MR-inaktiven, fiktiven Material ausgegangen werden, um die Signalveränderungen bzw. die Ausdehnungsgrenze um den Markerkern herum zu ermitteln bzw. abzuschätzen. Falls jedoch genauere Ermittlungen stattfinden sollen, ist es zweckmäßig, das Material der Markerhülle genau zu kennen und gegebenenfalls entsprechende Referenzmessungen bzw. Simulationen durchzuführen, bei denen die exakten Materialeigenschaften berücksichtigt sind. So kann beispielsweise die exakte Suszeptibilität des verwendeten Markerhüllenmaterials bei Referenzmessungen bzw. Simulationen berücksichtigt werden.

Bei einer weiteren Ausführungsform kann vorgesehen sein, dass bei dem Ermitteln der Signalveränderung eine Ausrichtung gegenüber einem Hauptmagnetfeld bei der Akquisition vorgegeben und bei dem Dimensionieren berücksichtigt wird. Die Signalveränderungen, die durch den Markerkern hervorgerufen werden, sind in der Regel nicht kugelsymmetrisch. Die Signalveränderungen werden nämlich in der Regel durch das Hauptmagnetfeld hervorgerufen, dessen Richtung vorgegeben ist. In der Regel ergibt sich daher eine Rotationssymmetrie um den Vektor des Hauptmagnetfelds und orthogonal zu dem Vektor des Hauptmagnetfelds allenfalls Punktsymmetrien um den Markerkern. Aufgrund dieser unterschiedlichen räumlichen Symmetrien ist es auch für die Herstellung des Markers günstig, wenn die Ausrichtung der Bildakquisition gegenüber dem Hauptmagnetfeld bekannt ist. Nur so kann sichergestellt werden, dass bestimmte Signaländerungen auch wirklich auf die Markerhülle begrenzt sind.

Erfindungsgemäß wird darüber hinaus bereitgestellt ein Marker für eine Akquisition mittels eines Magnetresonanztomographiesystems bei vorgegebener Feldstärke und vorgegebener Auslesebandbreite mit
- einer Markerhülle und
- einem Markerkern vorgegebener Größe und vorgegebenen Materials, der vollständig von der Markerhülle umgeben ist, wobei
- eine Dimension der Markerhülle derart gewählt ist, dass eine Ausdehnungsgrenze einer Signalveränderung gegenüber dem nicht vorhandenen Marker bei der Akquisition mit der vorgegebenen Feldstärke und der vorgegebenen Bandbreite um den Markerkern herum zumindest bereichsweise innerhalb der Markerhülle liegt, wobei innerhalb der Ausdehnungsgrenze die Signalveränderung einen vorgegebenen Betrag überschreitet.

Es wird also ein Marker bereitgestellt, bei dem die Signalveränderungen durch den Markerkern für die vorgegebene Feldstärke und die vorgegebene Auslesebandbreite zumindest bereichsweise innerhalb der Markerhülle auf einen akzeptablen Wert abgesunken sind. Vorzugsweise ist die Signalveränderung betragsmäßig in allen Raumrichtungen innerhalb der Markerhülle auf den gewünschten Wert abgesunken. Der Marker kann nach dem oben beschriebenen Verfahren bzw. dessen Weiterentwicklungen hergestellt sein.

In einer diesbezüglichen Weiterbildung ist vorgesehen, dass für die Markerhülle ein Material gewählt wird, dessen Relaxationszeit T1 bei der vorgegebenen Feldstärke unter 0,5s liegt oder das mit schwerem Wasser durchsetzt ist. Durch die relativ geringe Relaxationszeit baut sich in kurzer Zeit eine verhältnismäßig hohe Signalamplitude wieder auf. Dies hat zur Folge, dass der gesamte Marker, also auch die Markerhülle auf dem MR-Bild gut zu sehen ist. Falls beispielsweise die Markerhülle aus einem geschlossen-porigen Material besteht, das mit schwerem Wasser gefüllt ist, ergeben sich auch für das Markermaterial kurze Relaxationszeiten T1, so dass die Markerhülle auf den gewonnenen MR-Bildern gut erkennbar ist.

In einem weiteren Ausführungsbeispiel enthält der Markerkern Luft oder ist magnetisierbar. In beiden Fällen ist er dadurch in menschlichem Gewebe gut sichtbar.

Erfindungsgemäß wird aber auch ein Verfahren zur Akquisition eines Bildes mittels eines Magnetresonanztomographiesystems bereitgestellt. Es erfolgt zunächst ein Empfangen von MR-Signalen, welche in Bezug auf einen Marker, wie er oben beschrieben ist, analysiert werden. Das Analysieren erfolgt automatisch mit einem Mustererkennungsalgorithmus. Der Mustererkennungsalgorithmus wird auf die Feldstärke, eine Ausrichtung gegenüber einem Hauptmagnetfeld der Akquisition die Größe des Markerkerns, das Material des Markerkerns und/oder die Auslesebandbreite bei der Bildakquisition abgestimmt. Dabei kann der Mustererkennungsalgorithmus auf einen oder mehrere der oben genannten Parameter abgestimmt sein. Die Leistungsfähigkeit des Mustererkennungsalgorithmus steigt, wenn er auf immer mehr dieser Parameter abgestimmt wird. Bei einem einfachen Mustererkennungsalgorithmus kann es aber auch ausreichen, ihn beispielsweise nur auf die Feldstärke oder nur auf die Feldstärke und die Größe des Markerkerns abzustimmen, wenn andere Parameter beispielsweise vorgegeben sind. So kann beispielsweise die Ausrichtung des Hauptmagnetfelds vorgegeben sein, so dass es nicht mehr nötig ist, bei dem Akquisitionsverfahren die Ausrichtung individuell einzustellen.

Bei einer Weiterentwicklung dieses Akquiseverfahrens kann es vorgesehen sein, dass der Mustererkennungsalgorithmus auf eine Datenbank, eine Simulation und/oder eine selbstlernende Struktur zugreift. Aus einer Datenbank können zusätzliche Informationen wie Referenzbilder, Look-up-Tabellen und dergleichen gewonnen werden. Auch bei einer Simulation kann ein Referenzbild gewonnen werden, um dieses für eine Bildauswertung zugrunde zu legen. Auf diese Weise kann beispielsweise die Belastung des Patienten reduziert werden. Des Weiteren kann der Mustererkennungsalgorithmus eine selbstlernende Struktur wie etwa ein neuronales Netz nutzen. Hierdurch ist es möglich, Bildelemente besser zu klassifizieren und die Klassifizierung zu individualisieren.

Speziell kann vorgesehen sein, dass ein Marker in dem akquirierten Bild markiert wird. Dies lässt sich beispielsweise auf zuverlässige Weise dadurch erreichen, dass der Mustererkennungsalgorithmus mithilfe eines neuronalen Netzes einen Bildausschnitt als Marker klassifiziert und infolgedessen eine entsprechende Markierung an der Stelle des Markers gesetzt wird. Auf diese Weise kann der Anwender den Marker noch zuverlässiger auffinden.

Weiterhin kann erfindungsgemäß ein Computerprogrammprodukt vorgesehen sein, welches direkt in einen Speicher einer Steuereinrichtung eines Magnetresonanztomographiesystems ladbar ist, mit Programmmitteln, um die Schritte des oben genannten Verfahrens auszuführen, wenn das Programm in der Steuereinrichtung des Magnetresonanztomographiesystems ausgeführt wird. Das hierin beschriebene Verfahren kann also auch in Form eines Computerprogrammprodukts vorliegen, das das Verfahren auf einer Steuereinheit implementiert, wenn es auf der Steuereinheit ausgeführt wird. Ebenso kann ein elektronisch lesbarer Datenträger mit darauf gespeicherten elektronisch lesbaren Steuerinformationen vorliegen, welche zumindest ein beschriebenes Computerprogrammprodukt umfassen und derart ausgestaltet sind, dass sie bei der Verwendung des Datenträgers in einer Steuereinheit einer MR-Anlage ein beschriebenes Verfahren durchführen.

Die vorliegende Erfindung wird nun anhand der beigefügten Zeichnungen näher erläutert, in denen zeigen:
- FIG 1: einen Querschnitt durch einen Marker für ein Magnetresonanztomographiesystem;
- FIG 2: ein MR-Bild des Markers von FIG 1 und
- FIG 3: ein schematisches Blockdiagramm eines Verfahrens zur Herstellung eines Markers für ein Magnetresonanztomographiesystem.

Die nachfolgend näher geschilderten Ausführungsbeispiele stellen bevorzugte Ausführungsformen der vorliegenden Erfindung dar.

Bei vielen Anwendungen kann es wünschenswert sein, Marker bzw. Seeds verwenden zu können, die sowohl einfach sichtbar gemacht werden können als auch keine Artefakte in ihrem Umfeld hervorrufen. Zum anderen wäre es aber auch erstrebenswert, ein Verfahren zu finden, mit dem die Marker bzw. Seeds zuverlässig sichtbar gemacht werden können.

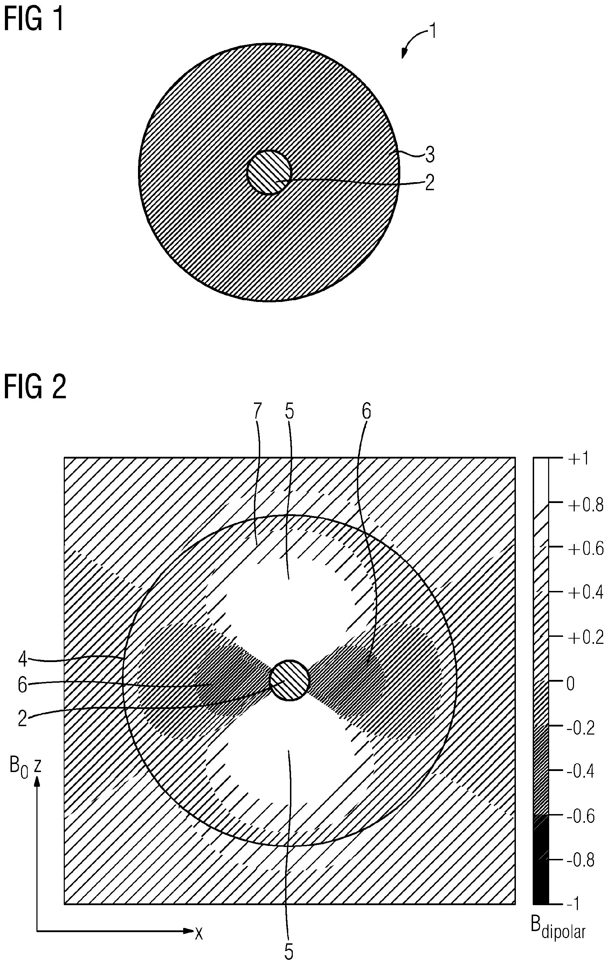

FIG 1 zeigt ein Ausführungsbeispiel eines Markers für ein Magnetresonanztomographiesystem im Querschnitt. Der Marker 1 ist hier kugelförmig ausgebildet. Beispielsweise besitzt er eine Größe zwischen 0,1 und 0,5cm. Dieses Maß entspricht hier also dem Außendurchmesser. Speziell besitzt der Marker 1 einen Markerkern 2 im Zentrum der Kugel und eine Markerhülle 3, die den Markerkern 2 hier vollständig umgibt.

Grundsätzlich muss ein Marker aber nicht kugelförmig sein. Vielmehr kann er beispielsweise auch ellipsoidförmig sein. Der Marker kann aber auch beispielsweise scheibenförmig sein, wenn sichergestellt werden kann, dass er immer in einer bestimmten Lage z.B. senkrecht zu einer Aufnahmerichtung bzw. senkrecht zu dem Hauptmagnetfeld platziert werden kann. Gegebenenfalls kann der Marker aber auch würfelförmig oder pyramidenförmig, quaderförmig oder dergleichen sein.

Der vorgeschlagene Marker 1 soll eine Suszeptibilitätsquelle (Markerkern 2) aufweisen, die jedoch so ausgestaltet bzw. angeordnet ist, dass ihre MR-sichtbare Wirkung bei einer aktuell verwendeten Feldstärke und einer aktuell verwendeten Auslesebandbreite innerhalb des Markers, d. h. innerhalb der Markerhülle 3 liegt. Dazu ist der Markerkern 2 als Suszeptibilitätsquelle in die Markerhülle 3 als umgebenden Stoff derart eingebettet, dass er außerhalb der Markerhülle keine sichtbaren MR-Störungen hervorruft.

Der Markerkern 2 kann beispielsweise bei medizinischen Anwendungen 0,5-1mm groß sein. Seine Größe kann bei Bedarf aber auch anders gewählt werden. Insbesondere kann es beispielsweise bei technischen Materialuntersuchungen auch günstig sein, den Markerkern deutlich größer oder deutlich kleiner zu wählen. Als Materialien für den Markerkern 2 kommen beispielsweise Stoffe in Frage, die schwach magnetisierbar sind. Hierunter fallen beispielsweise einige Metalle. Alternativ kann der Markerkern aber auch beispielsweise ein Lufteinschluss in der Markerhülle 3 sein. Auch so ein Lufteinschluss lässt sich auf einem MR-Bild als z.B. dunkler Punkt zuverlässig erkennen.

Die Markerhülle 3, also der den Markerkern 2 umgebende Stoff kann vorteilhafterweise so ausgelegt sein, dass er ein hohes MR-Signal liefert, indem er beispielsweise eine kurze Relaxationszeit T1 besitzt oder auch mit schwerem Wasser (D2O) durchsetzt ist. Bei kurzer Relaxationszeit T1 baut sich das MR-Signal rasch wieder auf und der Marker ist in dem MR-Bild gut sichtbar. Beispielsweise kann die Markerhülle 3 aus Agarose bestehen, die keine MR-Störungen hervorruft.

Der Markerkern wird in der Regel gerade wegen seiner guten Sichtbarkeit auch Artefakte bzw. Störungen hervorrufen, wie dies beispielhaft in FIG 2 dargestellt ist. In dieser Figur ist die Außengrenze 4 des Markers 1 kenntlich gemacht, ebenso wie der Markerkern 2. Es ergeben sich keulenförmige Störungen 5, 6, um den Markerkern 2 herum. Die Störungen 5 stellen im vorliegenden Beispiel Signalerhöhungen dar und sind heller als die Umgebung dargestellt. Demgegenüber stellen die keulenförmigen Störungen 6 Signalverminderungen dar und sind daher in der Figur dunkel dargestellt.

An der rechten Skala in FIG 2 ist die Stärke des magnetischen Dipolfelds B_{dipolar} dargestellt. Signalverminderungen werden dementsprechend dunkel wiedergegeben, während Signalerhöhungen hell dargestellt sind.

Weiterhin ist in FIG 2 die Richtung des Hauptmagnetfelds B₀ dargestellt. Das Hauptmagnetfeld B₀ weist in z-Richtung, also in der Figur nach oben. Orthogonal hierzu ist die x-Richtung eingezeichnet. Die Störungen 5, 6 sind also nicht rotationssymmetrisch, sondern ergeben sich aus der Richtung des Hauptmagnetfelds B₀. Im vorliegenden Beispiel liegen also die Signalerhöhungen 5 in z-Richtung oberhalb und unterhalb des Markerkerns 2, während die Signalverminderungen 6 links und rechts des Markerkerns 2 keulenförmig ausgebildet sind.

Es ist zu erkennen, dass eine Ausdehnungsgrenze 7 der größten Störung 5 in der Markerhülle 3, d. h. innerhalb der Außengrenze 4 liegt. Diese Ausdehnungsgrenze 7 stellt eine Sichtbarkeitsgrenze der Störung 5 dar. Beispielsweise liegt die Sichtbarkeitsgrenze in etwa bei einer Signalerhöhung um 20%. In diesem Fall würde die Sichtbarkeitsgrenze also durch eine Signaländerung von 20% liegen. Unter dieser Schwelle wäre die Änderung nicht oder nicht gut sichtbar. Dies bedeutet, dass in diesem Beispiel der vorgegebene Betrag, der als Schwelle für das Festlegen der Ausdehnungsgrenze der Signalveränderung vorgegeben wird, bei 20% liegt.

In dem Beispiel von FIG 2 wird die Ausdehnungsgrenze 7 in etwa durch zwei übereinanderliegende "8", die um 90° verdreht sind, gebildet. Innerhalb der im Bild senkrecht stehenden "8" befinden sich die Signalerhöhungen 5 und innerhalb der horizontal liegenden "8" befinden sich die Signalverminderungen 6. Während die Signalerhöhungen 5 hell dargestellt sind, sind die Signalverminderungen 6 dunkel dargestellt.

Die Dimension der Markerhülle 3, nämlich ihr Radius bzw. Durchmesser (Außengrenze 4) ist bei dem Marker von FIG 2 so gewählt, dass er größer ist als der radiale Abstand der Ausdehnungsgrenze 7 von dem Zentrum des Markerkerns 2. Dies äußert sich dadurch, dass die sichtbaren Signalveränderungen 5 und 6 in der Markerhülle 3 bzw. innerhalb der Außengrenze 4 liegen.

Je nach Wahl von Größe und Substanz der Suszeptibilitätsquelle bzw. des Markerkerns 2 kann die Ausweitung der Größe der Störung (Ausdehnungsgrenze 7) im MR-Bild verändert werden. Diese Ausweitung bzw. Ausdehnungsgrenze 7 hängt neben der Substanz auch von der Feldstärke, der Orientierung der Akquisition bzw. des Hauptmagnetfelds B₀ und der verwendeten Auslesebandbreite ab. Die Abhängigkeit von der Auslesebandbreite ist einleuchtend, da die Auslösung des MR-Bilds von der Auslesebandbreite abhängt und bei gröberer Auflösung die Störungen durchaus weiter nach außen reichen können.

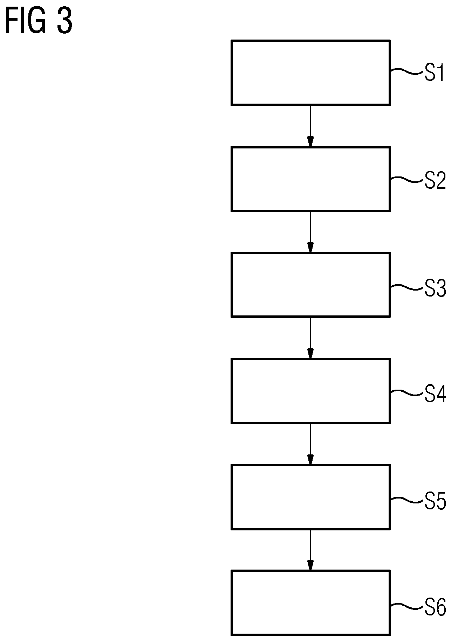

Die Herstellung eines Markers für die Magnetresonanztomographie kann gemäß dem schematischen Diagramm von FIG 3 erfolgen. In einem ersten Schritt S1 erfolgt ein Vorgeben einer Feldstärke für eine Akquisition mit dem Magnetresonanztomographiesystem. Es muss also definiert werden, für welche Feldstärke bzw. welchen Feldstärkebereich der Marker verwendet werden soll.

In einem zweiten Schritt S2 erfolgt ein Vorgeben einer Auslesebandbreite bei der Akquisition mit dem Magnetresonanztomographiesystem. Auch hier wird im Vorfeld der Herstellung festgelegt, wie groß die Auslesebandbreite bei der Akquisition sein soll. Gegebenenfalls kann auch hier ein gewisser Bereich festgelegt werden, innerhalb dessen die Auslesebandbreite liegt. Die Auslesebandbreite wird oftmals auch als Auslesebandweite des MR-Scanners bezeichnet.

In einem weiteren Schritt S3 wird eine Größe und ein Material für den Markerkern des Markers vorgegeben. Dies bedeutet, dass vor der Herstellung natürlich die Abmessungen und das Material für den Markerkern festgelegt werden müssen. Vorzugsweise handelt es sich bei dem Markerkern um eine Kugel, sie kann aber auch eine andere Gestalt, wie etwa ein Stern, ein Würfel, ein Stab oder dergleichen sein. Wenn also beispielsweise stets davon ausgegangen wird, dass der Markerkern kugelförmig ist, so muss im Rahmen des Herstellungsverfahrens nur sein Radius bzw. Durchmesser vorgegeben werden. Wenn allerdings die Gestalt des Markerkerns generell offen ist, muss auch die Form des Markerkerns vorgegeben werden.

In einem weiteren Schritt S4 wird eine Signalveränderung gegenüber dem nicht vorhandenen Marker bei der Akquisition mit der vorgegebenen Feldstärke und der vorgegebenen Bandbreite um den Markerkern herum ermittelt. Es werden also die in den Schritten S1-S3 festgelegten bzw. gewonnenen Parameter genutzt, um zu analysieren, wie sie sich auf das Signal bei der Bild-Akquisition auswirken. Dieses Ermitteln der Signalveränderung kann durch Messungen erfolgen, aber auch durch Simulationen.

In einem weiteren Schritt S5 wird die Ausdehnungsgrenze 7 um den Markerkern 2 herum ermittelt. Dabei kann das Augenmerk darauf gelegt werden, wie weit die Signalveränderung in dem MR-Bild sichtbar ist. Diese Sichtbarkeitsgrenze einer Signalveränderung kann aber auch beispielsweise durch Erfahrung vorgegeben werden, indem beispielsweise ein entsprechender Betrag für die Signalveränderung festgelegt wird, wie dies oben erläutert wurde. Die Ausdehnungsgrenze 7 wird also so ermittelt, dass die Signalveränderung (vorzugsweise jede durch den Markerkern hervorgerufene Signalveränderung) den vorgegebenen Betrag an der jeweiligen Stelle über- bzw. unterschreitet. Die stärksten Signalveränderungen erfolgen in der Nähe des Markerkerns 2. Mit zunehmendem Abstand von dem Markerkern 2 nehmen auch die Signalveränderungen ab. Dieses Abnehmen erfolgt allerdings nicht kugelsymmetrisch, sondern ist durch die Richtung des Hauptmagnetfelds B₀ bedingt, wie dies im Zusammenhang mit FIG 2 erläutert wurde.

Die Ausdehnungsgrenze 7 muss nicht vollständig und zusammenhängend sein. Vielmehr genügt es, wenn beispielsweise Maximalentfernungen von dem Markerkern 2 festgelegt werden. In dem Beispiel von FIG 2 genügt es beispielsweise, wenn die Außengrenze der Signalerhöhungen 5 auf der z-Achse durch den Markerkern 2 ermittelt wird, denn die größten Signalerhöhungen 5 sind in Richtung des Hauptmagnetfelds B₀ zu erwarten. Gegebenenfalls können auch noch die Maximalwerte der Signalverminderungen 6 auf der x-Achse durch den Markerkern 2 ermittelt werden. Unter Umständen reicht es aber aus Symmetriegründen und der Kenntnis über Signalerhöhungen 5 und Signalverminderungen 6, dass im Rahmen des Schritts S5 lediglich ein einziger Wert für die Ausdehnungsgrenze 7 ermittelt wird, nämlich der größte Abstand der Signalerhöhung 5 auf der z-Achse.

In einem weiteren Schritt S6 erfolgt das Dimensionieren der Markerhülle 3 des Markers 1 um den Markerkern 2 derart, dass die Ausdehnungsgrenze 7 zumindest bereichsweise innerhalb der Markerhülle 3 liegt. Die sichtbare Signalveränderung soll sich also nicht über die Markerhülle 3 hinaus erstrecken, wie dies in FIG 2 angedeutet ist. Das Dimensionieren der Markerhülle 3 kann beispielsweise dadurch erfolgen, dass bei einer Referenzmessung mit einem Marker, der eine übergroße Markerhülle besitzt, die Ausdehnungsgrenze ermittelt wurde und nun der Radius der Markerhülle für die Herstellung weiterer Marker exakt auf die Ausdehnungsgrenze gesetzt wird. Beispielsweise kann der Außenradius des Markers also so gewählt werden, dass er der Entfernung vom Markerzentrum bis zur Ausdehnungsgrenze auf der z-Achse entspricht. Bei anders geformten Markern kann die Dimensionierung der Markerhülle auch auf andere Weise in Abhängigkeit von der Ausdehnungsgrenze 7 erfolgen. In jedem Fall sollte beachtet werden, dass die Ausdehnungsgrenze 7 zumindest bereichsweise, vorzugsweise vollständig, innerhalb der Markerhülle 3 liegt.

Die Marker bzw. Seeds können in den MR-Bildern auf bekannte Art und Weise sichtbar gemacht werden. In den folgenden Artikeln sind entsprechende Verfahren beschrieben:
- Seppenwoolde, J.H., M.A. Viergever, and C.J. Bakker, Passive tracking exploiting local signal conservation: the white marker phenomenon. Magn Reson Med, 2003. 50(4): p. 784-90.
- Mani, V., et al., Gradient echo acquisition for superparamagnetic particles with positive contrast (GRASP): sequence characterization in membrane and glass superparamagnetic iron oxide phantoms at 1.5T and 3T. Magn Reson Med, 2006. 55(1): p. 126-35
- Stuber, M., et al., Positive contrast visualization of iron oxide-labeled stem cells using inversion-recovery with ON-resonant water suppression (IRON). Magn Reson Med, 2007. 58(5): p. 1072-7.

Diese oben beschriebenen Verfahren können aber durch das im Folgenden beschriebene Verfahren ergänzt oder ersetzt werden. Mit diesem Verfahren sind eine einfache Erkennung und Lokalisierung eines Markers möglich. Das Verfahren beruht darauf, dass ein automatisierter Mustererkennungsalgorithmus auf das MR-Bild angewendet wird, der die zu erwartenden Störungen im Bild erkennt. Dabei berücksichtigt der Algorithmus die folgenden Aspekte, die die Störungen in Größe und Form beeinflussen:
- Feldstärke des verwendeten MR-Scanners;
- Ausrichtung der Akquisition;
- verwendete Bandbreite, Auflösung und Schichtdicken für die Aufnahme;
- Eigenschaften der Seeds (Größe und Informationen wie Stärke des Dipolfelds über die Suszeptibilitätsquelle).

Der Algorithmus kann die Seeds und die für die genannten Aspekte erwarteten Störungen erkennen, indem er beispielsweise auf eine Datenbank zurückgreift, in der die Informationen der oben genannten Aspekte gespeichert sind. Alternativ oder zusätzlich kann der Algorithmus auch die Störungen anhand einer Simulation erkennen, die die genannten Aspekte berücksichtigt und die Störungen berechnet. Weiterhin kann der Algorithmus auch auf sogenanntes Deep-Learning gestützt sein und in einer Trainingsphase mit hinreichend Beispielen geschult werden.

Schließlich kann der Algorithmus auch so ausgelegt werden, dass er dem Benutzer oder einem weiterführenden Algorithmus die gefundenen Seeds im Bild markiert.

## Patentansprüche

1. Verfahren zum Herstellen eines Markers (1) für ein Magnetresonanztomographiesystem
**gekennzeichnet durch**
- Vorgeben (S1) einer Feldstärke für eine Akquisition mit dem Magnetresonanztomographiesystem,
- Vorgeben (S2) einer Auslesebandbreite bei der Akquisition mit dem Magnetresonanztomographiesystem,
- Vorgeben (S3) einer Größe und eines Materials für einen Markerkern (2) des Markers (1) und
- Ermitteln (S4) einer Signalveränderung (5, 6) gegenüber dem nicht vorhandenen Marker bei der Akquisition mit der vorgegebenen Feldstärke und der vorgegebenen Bandbreite um den Markerkern (2) herum,
- Ermitteln (S5) einer Ausdehnungsgrenze (7) um den Markerkern (2) herum, innerhalb der die Signalveränderung (5, 6) einen vorgegebenen Betrag überschreitet, und
- Dimensionieren (S6) einer Markerhülle (3) des Markers (1) um den Markerkern (2) derart, dass die Ausdehnungsgrenze (7) zumindest bereichsweise innerhalb der Markerhülle (3) liegt.

2. Verfahren nach Anspruch 1, wobei ein Material für die Markerhülle (3), die den Markerkern (2) vollständig umgibt, vorgegeben und bei dem Ermitteln der Signalveränderung (5, 6) berücksichtigt wird.

3. Verfahren nach Anspruch 1 oder 2, wobei bei dem Ermitteln der Signalveränderung (5, 6) eine Ausrichtung gegenüber einem Hauptmagnetfeld (B₀) bei der Akquisition vorgegeben und bei dem Dimensionieren berücksichtigt wird.

4. Marker (1) für eine Akquisition mittels eines Magnetresonanztomographiesystems bei vorgegebener Feldstärke und vorgegebener Auslesebandbreite mit
- einer Markerhülle (3) und
- einem Markerkern (2) vorgegebener Größe und vorgegebenen Materials, der vollständig von der Markerhülle (3) umgeben ist,
**dadurch gekennzeichnet, dass**
- eine Dimension der Markerhülle (3) derart gewählt ist, dass eine Ausdehnungsgrenze (7) einer Signalveränderung (5, 6) gegenüber dem nicht vorhandenen Marker bei der Akquisition mit der vorgegebenen Feldstärke und der vorgegebenen Bandbreite um den Markerkern (2) herum zumindest bereichsweise innerhalb der Markerhülle (3) liegt, wobei innerhalb der Ausdehnungsgrenze (7) die Signalveränderung (5, 6) einen vorgegebenen Betrag überschreitet.

5. Marker (1) nach Anspruch 4, wobei für die Markerhülle (3) ein Material gewählt ist, dessen Relaxationszeit T1 bei der vorgegebenen Feldstärke unter 0,5s liegt oder das mit schwerem Wasser durchsetzt ist.

6. Marker (1) nach Anspruch 4 oder 5, wobei der Markerkern (2) magnetisierbar ist oder Luft enthält.

7. Verfahren zur Akquisition eines Bildes mittels eines Magnetresonanztomographiesystems durch
- Empfangen von MR-Signalen,
**gekennzeichnet durch**
- Analysieren der MR-Signale in Bezug auf einen Marker (1) gemäß Anspruch 4, 5 oder 6, wobei
- das Analysieren automatisch mit einem Mustererkennungsalgorithmus erfolgt,
- der Mustererkennungsalgorithmus auf die Feldstärke, eine Ausrichtung gegenüber einem Hauptmagnetfeld der Akquisition, die Größe des Markerkerns (2), das Material des Markerkerns (2) und/oder die Auslesebandbreite abgestimmt wird.

8. Verfahren nach Anspruch 7, wobei der Mustererkennungsalgorithmus auf eine Datenbank, eine Simulation und/oder eine selbstlernende Struktur zugreift.

9. Verfahren nach Anspruch 7 oder 8, wobei der Marker (1) in dem akquirierten Bild markiert wird.

10. Computerprogrammprodukt, welches direkt in einen Speicher einer Steuereinrichtung eines Magnetresonanztomographiesystems ladbar ist, mit Programmmitteln, um die Schritte des Verfahrens nach einem der Ansprüche 7 bis 9 auszuführen, wenn das Programm in der Steuereinrichtung des Magnetresonanztomographiesystems ausgeführt wird.
